# EUROPÄISCHE PATENTSCHRIFT

(11) **EP 1 189 615 B1**
(45) Veröffentlichungstag und Bekanntmachung des Hinweises auf die Patenterteilung: **04.02.2004**
(21) Anmeldenummer: 00940235.5
(22) Anmeldetag: 04.05.2000
(51) Int. Cl.: A61K 31/495, A61K 38/15, A61P 33/00

(54) **ENDOPARASITIZIDE SYNERGISTISCHE KOMBINATION ENTHALTEND CYCLISCHE DEPSIPEPTIDE UND PIPERAZINE**
ENDOPARASITICIDAL SYNERGISTIC COMBINATION CONTAINING CYCLIC DEPSIPEPTIDES AND PIPERAZINES
COMBINAISON ENDOPARASITICIDE SYNERGIQUE CONTENANT DES DEPSIPEPTIDES CYCLIQUES ETDES PIPERAZINES

(30) Priorität: 12.05.1999 DE 19921887
(43) Veröffentlichungstag der Anmeldung: 27.03.2002
(73) Patentinhaber: Bayer HealthCare AG, 51368 Leverkusen (DE)
(72) Erfinder: HARDER, Achim, D-51109 Köln (DE); VON SAMSON-HIMMELSTJERNA, Georg, D-42657 Solingen (DE)
(86) Internationale Anmeldenummer: PCT/EP2000/004014
(87) Internationale Veröffentlichungsnummer: WO 2000/069425

(56) Entgegenhaltungen:
- EP-A- 0 718 293
- EP-A- 1 028 126
- WO-A-97/09331
- WO-A-98/37088
- DE-A- 19 520 275
- US-A- 5 589 503
- ZA-A- 8 402 571
- VON SAMSON-HIMMELSTJERNA, G. (1) ET AL: "The effect of the cyclic depsipeptide Bay 44-44 is synergistically enhanced by the GABA agonist piperazine - indicating a new neuropharmacological action." EUROPEAN JOURNAL OF NEUROSCIENCE, (2000) VOL. 12, NO. SUPPLEMENT 11, PP. 43. PRINT. MEETING INFO.: MEETING OF THE FEDERATION OF EUROPEAN NEUROSCIENCE SOCIETIES BRIGHTON, UK JUNE 24-28, 2000 , XP000964691
- PATENT ABSTRACTS OF JAPAN vol. 013, no. 275 (C-610), 23. Juni 1989 (1989-06-23) & JP 01 071865 A (NICHIBAI BOEKI KK;OTHERS: 01), 16. März 1989 (1989-03-16)
- GEARY T G ET AL: "Frontiers in anthelmintic pharmacology." VETERINARY PARASITOLOGY, (1999 AUG 1) 84 (3-4) 275-95. REF: 98 , XP000961795

## Beschreibung

Die vorliegende Erfindung betrifft die Verwendung von Piperazinen zur Steigerung der endoparasitiziden Wirkung von cyclischen Depsipeptiden in endoparasitiziden Mitteln.

Piperazine und ihre Wirkung gegen Endoparasiten sind allgemein bekannt. (Mehlhom et al., Diagnostik und Therapie der Parasitosen des Menschen, 2. Auflage, Gustav Fischer Verlag, (1995), Mehlhorn et al., Diagnostik und Therapie der Parasitosen von Haus-, Nutz- und Heimtieren, 2. Auflage, Gustav Fischer Verlag, (1993).)

Ein cyclisches Depsipeptid PF 1022 und seine Wirkung gegen Endoparasiten ist bekannt aus EP-OS 382 173.

Weitere cyclische Depsipeptide und ihre endoparasitizide Wirkung sind Gegenstand von EP-OS 0 626 375, EP-OS 0 626 376 und WO 93/25543.

Es wurde nun gefunden, dass Piperazine verwendet werden können zur Steigerung der endoparasitiziden Wirkung von cyclischen Depsipeptiden bestehend aus Aminosäuren und Hydroxycarbonsäuren als Ringbausteinen und 24 Ringatomen.

Gegenstand der vorliegenden Erfindung sind ferner endoparasitizide Mittel, die Piperazine zusammen mit cyclischen Depsipeptiden bestehend aus Aminosäuren und Hydroxycarbonsäuren als Ringbausteinen und 24 Ringatomen enthalten.

Gegenstand der vorliegenden Erfindung ist ferner die Verwendung von Piperazinen zusammen mit cyclischen Depsipeptiden bestehend aus Aminosäuren und Hydroxycarbonsäuren als Ringbausteinen und 24 Ringatomen zur Herstellung von endoparasitiziden Mitteln.

Zu den cyclischen Depsipeptiden mit 24 Ringatomen zählen Verbindungen der allgemeinen Formel (I) 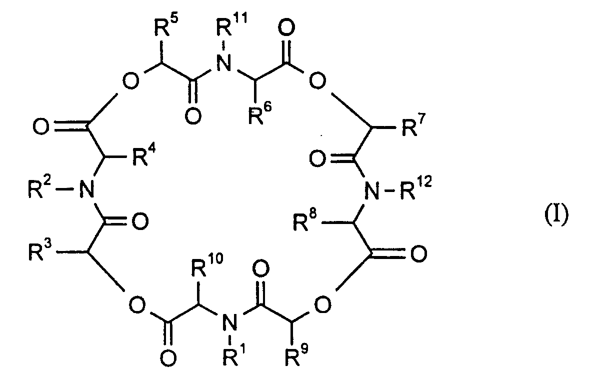 in welcher
- R¹, R², R¹¹ und R¹²: unabhängig voneinander für C₁₋₈-Alkyl, C₁₋₈-Halogenalkyl, C₃₋₆-Cycloalkyl, Aralkyl, Aryl stehen,
- R³, R⁵, R⁷, R⁹: unabhängig voneinander für Wasserstoff oder geradkettiges oder verzweigtes C₁₋₈-Alkyl steht, das gegebenenfalls durch Hydroxy, C₁₋₄-Alkoxy, Carboxy, 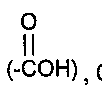 Carboxamid, 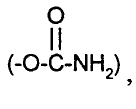 , Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
- R⁴, R⁶, R⁸, R¹⁰: unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen sowie deren optische Isomere und Racemate.

Bevorzugt werden Verbindungen der Formel (I) eingesetzt, in welcher
R¹, R², R¹¹ und R¹² unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können und
R³ bis R¹⁰ die oben angegebene Bedeutung haben.

Besonders bevorzugt sind Verbindungen der Formel (I), in welcher
- R¹, R², R¹¹ und R¹²: unabhängig voneinander für Methyl, Ethyl, Propyl, Isopropyl oder n-, s-, t-Butyl stehen,
- R³, R⁵, R⁷, R⁹: für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können und
- R⁴, R⁶, R⁸, R¹⁰: unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können sowie für Isopropyl, s-Butyl ferner für gegebenenfalls halogensubstituiertes Phenyl, Benzyl oder Phenylethyl stehen.

Weiterhin sei als 24 ringgliedriges Depsipeptid die aus EP-OS 382 173 bekannte Verbindung PF 1022 der folgenden Formel genannt:

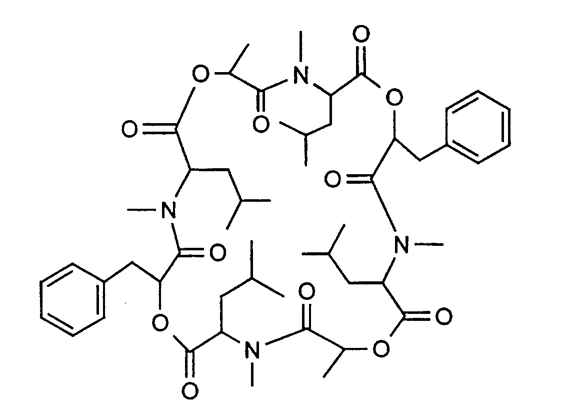

Außerdem seien als Depsipeptide die aus der PCT-Anmeldung WO 93/19053 bekannten Verbindungen genannt.

Insbesondere seien aus PCT-Anmeldung WO 93/19053 die Verbindungen der folgenden Formel genannt: 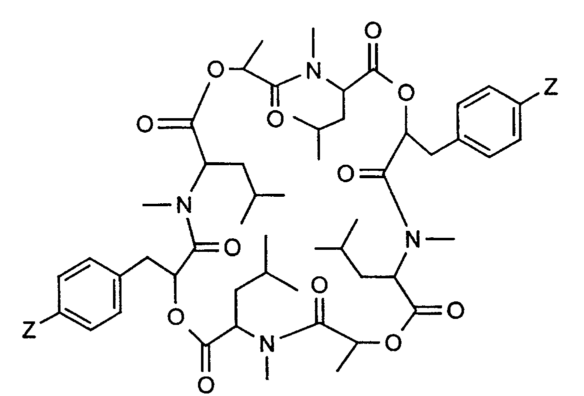 in welcher
- Z: für Morpholinyl, Nitro, Amino, Mono- oder Dimethylamino steht, besonders hervorgehoben für Morpholinyl.

Außerdem seien Verbindungen der folgenden Formel genannt: 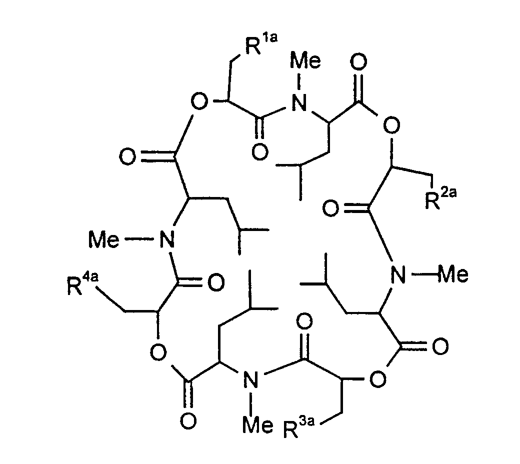 in welcher
- R^{1a}, R^{2a}, R^{3a}, R^{4a}: unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der Formel (I) können hergestellt werden, indem man offenkettige Octadepsipeptide der Formel (II) 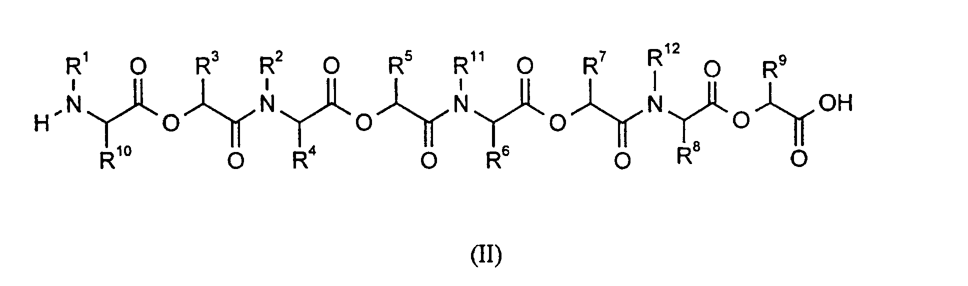 in welcher
R¹ bis R¹² die oben angegebene Bedeutung haben,
in Gegenwart eines Verdünnungsmittels und in Gegenwart eines Kupplungsreagenzes cyclisiert.

Als Kupplungsreagenzien eignen sich alle Verbindungen, die zur Knüpfung einer Amidbindung geeignet sind (vgl. z.B.: Houben-Weyl, Methoden der organischen Chemie, Band 15/2; Bodanszky et al., Peptide Synthesis 2nd ed. (Wiley / Sons, New York 1976).

Vorzugsweise kommen folgende Reagenzien und Methoden in Frage: Aktivestermethode mit Pentafluorphenol (Pfp), N-Hydroxysuccinimid, 1-Hydroxybenzotriazol, Kupplung mit Carbodiimiden, wie Dicyclohexylcarbodiimid oder N'-(3-Dimethylaminopropyl)-N-ethyl-carbodiimid (Ebc) sowie die gemischte Anhydrid-Methode oder die Kupplung mit Phosphoniumreagenzien, wie Benzotriazol-1-yl-oxytris(dimethylaminophosphonium)-hexafluorophosphat (BOP), Bis(2-oxo-3-oxazolidinyl)-phosphoniumsäurechlorid (BOP-Cl), oder mit Phosphonsäureesterreagentien, wie Cyanphosphonsäurediethylester (DEPC) und Diphenylphospharylazid (DPPA).

Besonders bevorzugt ist die Kupplung mit Bis(2-oxo-3-oxazolidinyl)-phosphoniumsäurechlorid (BOP-Cl) und N'-(3-Dimethylaminopropyl)-N-ethylcarbodiimid (EDC) in Gegenwart von 1-Hydroxybenzotriazol (HOBt).

Die Umsetzung erfolgt bei Temperaturen von 0 - 150°C, bevorzugt bei 20 bis 100°C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Verbindungen der Formeln (II) und die Kupplungsreagenzien werden im Verhältnis 1:1 bis 1:1,5 zueinander eingesetzt. Bevorzugt ist ein etwa äquimolares Verhältnis.

Nach erfolgter Umsetzung wird das Verdünnungsmittel abdestilliert und die Verbindungen der Formel (I) in üblicher Weise, z.B. chromatographisch, gereinigt.

Die offenkettigen Octadepsipeptide der Formel (II) 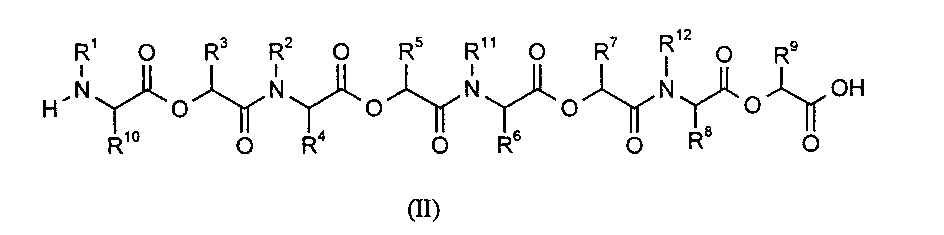 in welcher die Reste die oben angegebenen Bedeutungen haben werden erhalten, indem man Verbindungen der Formel (III) 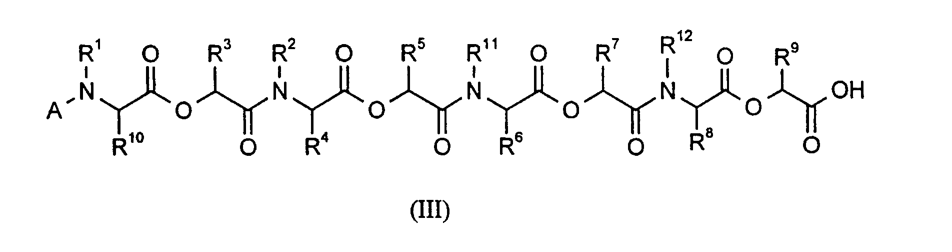 in welcher
- A: für Benzyl und
- R¹ bis R¹²: die oben angegebene Bedeutung besitzen,
in Gegenwart eines Verdünnungsmittels und eines Katalysators hydrogenolysiert.

Die Verbindungen der Formel (III) 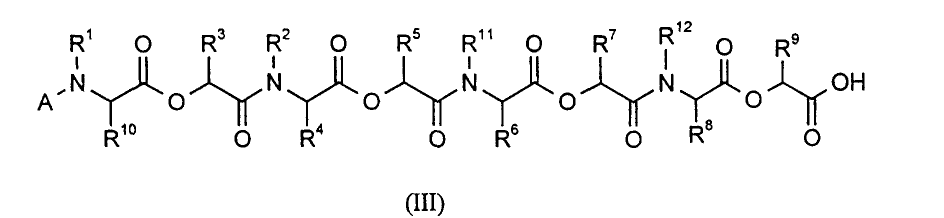 in welcher die Reste die oben angegebene Bedeutung haben, werden erhalten, indem man Verbindungen der Formel (IV) 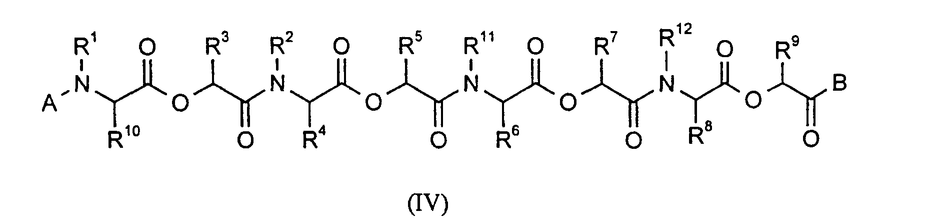 in welcher die Reste A und R¹ bis R¹² die oben angegebene Bedeutung haben und B für t-Butoxy steht, hydrolysiert.

Verbindungen der Formel (IV) sowie deren Stereoisomere, werden erhalten, indem man Tetradepsipeptide der Formel (V) 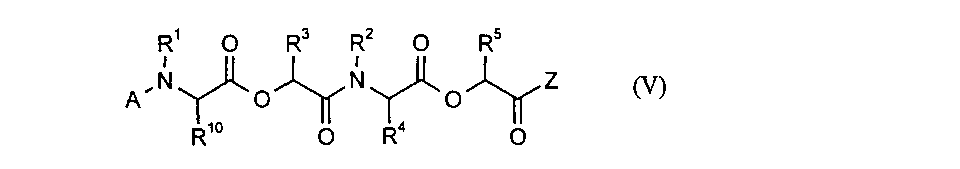 in welcher
- A: für Benzyl und
- Z: für OH steht sowie
- R¹, R², R³, R⁴, R⁵ und R¹⁰: die oben angegebene Bedeutung haben
und Tetradepsipeptide der Formel (VI) 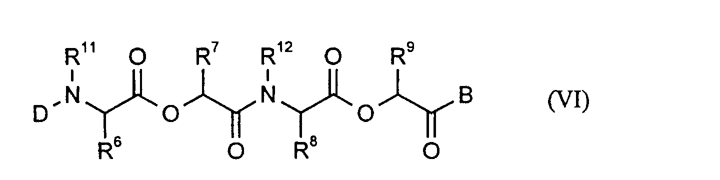 in welcher
- D: für Wasserstoff und
- B: für tert.-Butoxy steht sowie
- R⁶, R⁷, R⁸, R⁹, R¹¹ und R¹²: die oben angegebene Bedeutung haben,
in Gegenwart eines Verdünnungsmittels und eines Kupplungsreagenzen kondensiert.

Tetradepsipeptide der Formel (V) werden erhalten, indem man Tetradepsipeptide der Formel (VII) 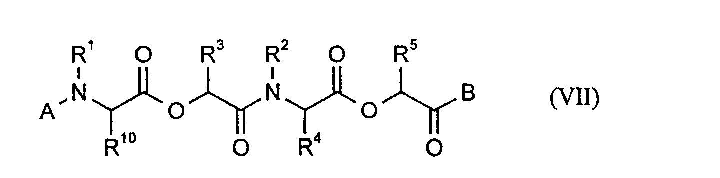 in welcher
- A: für Benzyl und
- B: für tert.-Butoxy steht sowie
- R¹, R², R³, R⁴, R⁵ und R¹⁰: die oben angegebene Bedeutung besitzen,
in Gegenwart eines Verdünnungsmittels und einer Protonensäure verseift.

Tetradepsipeptide der Formel (VI) 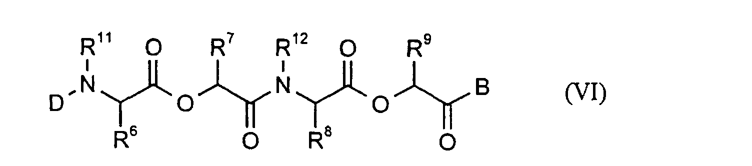 in welcher
- D: für Wasserstoff und
- B: für tert.-Butoxy steht und die übrigen Reste die oben angegebene Bedeutung haben,
werden erhalten, indem man Tetradepsipeptide der Formel (VII) 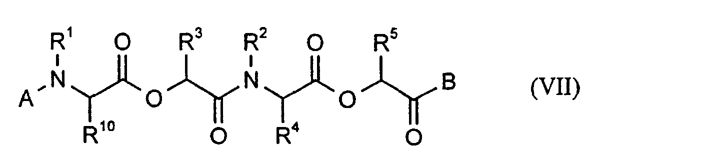 in welcher
- A: für Benzyl und
- B: für tert.-Butoxy steht sowie
- R¹, R², R³, R⁴, R⁵ und R¹⁰: die oben angegebene Bedeutung besitzen,
in Gegenwart eines Verdünnungsmittels und eines Katalysators hydrogenolysiert.

Tetradepsipeptide der Formel (VII) werden erhalten, indem man Didepsipeptide der Formel (VIII) 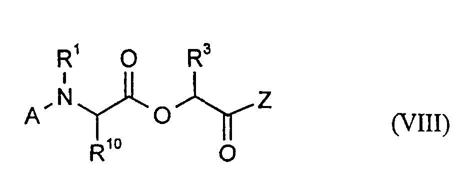 in welcher
- A: für Benzyl und
- Z: für OH steht sowie
- R¹, R³ und R¹⁰: die oben angegebene Bedeutung besitzen und

Didepsipeptide der Formel (IX) 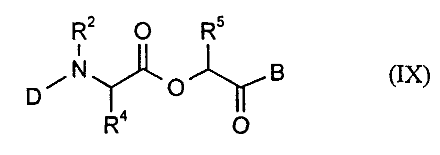 in welcher
- D: für Wasserstoff und
- B: für tert.-Butoxy steht sowie
- R², R⁴ und R⁵: die oben angegebene Bedeutung besitzen,
in einem Verdünnungsmittel in Gegenwart eines Kupplungsreagenzes kondensiert.

Die aus WO 93/19 053 bzw. aus EP-OS 382 173 bekannten Depsipeptide können nach den dort beschriebenen Methoden enthalten werden.

Zu den Piperazinen gehören alle Verbindungen der Formel (X) 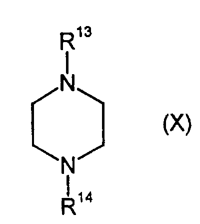 in welcher
- R¹³ und R¹⁴: unabhängig voneinander für gleiche oder verschiedene Substituenten aus der Gruppe Wasserstoff, jeweils gegebenenfalls substituiertes Alkyl, Cycloalkyl, Aryl, Heteroaryl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, jeweils gegebenenfalls substituiertes Alkyl oder Cycloalkyl stehen.

Bevorzugt sind Verbindungen der Formel (X) in welcher
- R¹³ und R¹⁴: unabhängig voneinander für gleiche oder verschiedene Substituenten aus der Gruppe Wasserstoff, jeweils gegebenenfalls substituiertes C₁-C₆-Alkyl, C₃-C₈-Cycloalkyl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, jeweils gegebenenfalls substituiertes C₁-C₆-Alkyl oder C₃-C₈-Cycloalkyl stehen.

Besonders bevorzugt sind Verbindungen der Formel (X) in welcher
- R¹³ und R¹⁴: unabhängig voneinander für gleiche oder verschiedene Substituenten aus der Gruppe Wasserstoff, jeweils gegebenenfalls substituiertes C₁-C₄-Alkyl, C₆-Cycloalkyl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, jeweils gegebenenfalls substituiertes C₁-C₄-Alkyl oder C₆-Cycloalkyl stehen.

Ganz besonders bevorzugt sind Verbindungen der Formel (X) in welcher
- R¹³ und R¹⁴: unabhängig voneinander für gleiche oder verschiedene Substituenten aus der Gruppe Wasserstoff, C₁-C₄ Alkyl, C₁-C₄-Haloalkyl mit 1 bis 9 gleichen oder verschiedenen Halogenatomen aus der Reihe F, Cl, oder Br, C₆-Cycloalkyl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, C₁-C₄-Alkyl oder C₆-Cycloalkyl stehen.

Beispielhaft, aber nicht einschränkend seien die folgenden Verbindungen genannt: Piperazin, Diethylcarbamazin, N,N'-Dimethylpiperazin, N-Methylpiperazin, N,N'-Diethylpiperazin, N-Ethylpiperazin, N-Ethyl-N'-methylpiperazin, N,N'-Dipropylpiperazin, N-Propylpiperazin, N-Ethyl-N'-propylpiperazin, N-Methyl-N'-propylpiperazin, N-Cyclohexylpiperazin, N,N'-Dicyclohexylpiperazin, 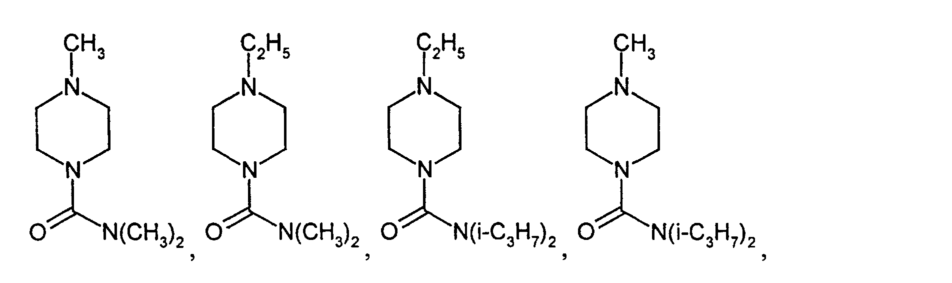 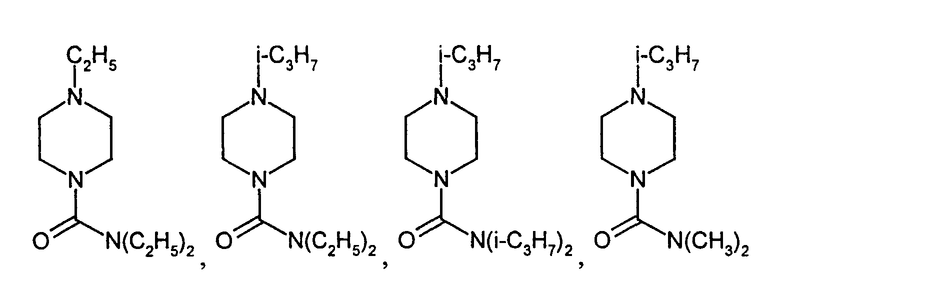 speziell hervorgehoben seien dabei Piperazin und Diethylcarbamazin.

Die Piperazine sind allgemein bekannte organische Verbindungen und sind kommerziell erhältlich oder können nach bekannten Methoden erhalten werden. (Mehlhorn et al. Diagnostik und Therapie der Parasitosen des Menschen 2. Auflage, Gustav Fischer (1995), Mehlhorn et al. Diagnostik und Therapie der Parasitosen von Haus-, Nutz- und Heimtieren, 2. Auflage Gustav Fischer (1993)).

Die erfindungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Omithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,

Globocephalus spp., Syngamus spp., Cyathostomum spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp., Cylicocyclus spp., Crateostonum spp., Cylicodontophorus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffmischungen erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffmischungen geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on und spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; halbfeste Zubereitungen;

Formulierungen bei denen die Wirkstoffmischung in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffmischungshaltige Formkörper.

Injektionslösungen werden intravenös, intramuskulär und subcutan verabreicht.

Injektionslösungen werden hergestellt, indem die Wirkstoffmischung in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffmischung lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung der Wirkstoffmischung im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem die Wirkstoffmischung in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man die Wirkstoffmischung entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C ₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

### Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervemetztes Polyvinylpyrrolidon, Bindemittel wie z.B. Stärke, Gelatine oder linerares Polyvinylpyrrolidon sowie Trockenbindemittel wie mikrokristalline Cellulose.

Die Wirkstoffmischungen können in den Zubereitungen auch in Mischung mit weiteren Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazothiazol, Benzimidazolcarbamate, Pyrantel.

Anwendungsfertige Zubereitungen enthalten die Wirkstoffmischungen in Konzentrationen von 10 ppm - 20 Gewichtsprozent, bevorzugt von 0,1-10 Gewichtsprozent.

Zubereitungen die vor Anwendung verdünnt werden, enthalten die Wirkstoffmischungen in Konzentrationen von 0,5-90 Gew.-%, bevorzugt von 5 bis 50 Gewichtsprozent.

Im allgemeinen hat es sich als vorteilhaft erwiesen, Mengen der erfindungsgemäßen Mischung von etwa 10 bis etwa 100 mg Wirkstoffmischung je kg Körpergewicht pro Tag zur Erzielung wirksamer Ergebnisse zu verabreichen. Bevorzugt sind 10 bis 50 mg Wirkstoffmischung je kg Körpergewicht.

In den Mitteln wird im allgemeinen ein Gewichtsverhältnis von Piperazin zu Depsipeptid wie 50:1 bis 1000:1, bevorzugt 100:1 bis 1000:1, ganz besonders bevorzugt 250:1 bis 1000:1, insbesondere 250:1 und 1000:1, eingehalten.

In den biologischen Beispielen wurde als "Depsipeptid I" die Verbindung der Formel 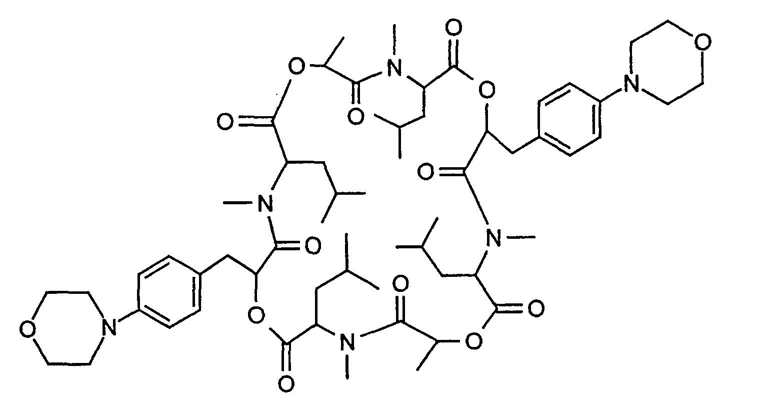 bekannt aus WO 93/19 053, eingesetzt.

Die Durchführung der biologischen Tests erfolgte entsprechend den bekannten Verfahren (Plant et. al. Pesticide Science, 1996, 48, S. 351 ff.).

### Biologische Beispiele

**Tabelle 1**

| Synergistischer Effekt von Piperazin und Depsipeptid I gegen Trichinella spiralis in-vitro | | |
|---|---|---|
| | Konzentration (µg/ml) | Wirkung |
| Piperazin | 1000 | 0-1 |
| | 500 | 0 |
| Depsipeptid I | 0,01 | 0-1 |
| | 0,001 | 0 |
| Piperazin/Depsipeptid I | 1000/0,01 | 1-2 |
| Piperazin/Depsipeptid I | 500/0,01 | 1-2 |
| 0 = keine Wirkung; 1 = schwache Wirkung; 2 = gute Wirkung | | |

**Tabelle 2**

| Synergistischer Effekt von Piperazin und Depsipeptid I gegen Nematoden der Maus | | | | | |
|---|---|---|---|---|---|
| Heterakis spumosa | Dosis (mg/kg) | Wirkung | Nematospiroides dibius | Dosis (mg/kg) | Wirkung |
| Piperazin | 4 x 250 | 1 | Piperazin | 2 x 2000 | 2 |
| | 4 x 100 | 0 | | 4 x 1000 | 1 |
| Depsipeptid I | 4 x 1 | 1 | PF1022-221 | 4 x 1 | 2 |
| | 4 x 0,5 | 1 | | 4 x 0,5 | 0-2 |
| Piperazin/ | 4 x 250/ | 3 | Piperazin/ | 2 x 2000/ | 2-3 |
| Depsipeptid I | 4 x 1 | | PF1022-221 | 4 x 1 | |
| Piperazin/ | 4 x 100/ | 2 | Piperazin/ | 4 x 1000/ | 2-3 |
| Depsipeptid 1 | 4 x 1 | | PF1022-221 | 4 x 1 | |
| 0 = Wurmreduktion <50 %; 1 = Wurmreduktion 50-75 %; 2 = Wurmreduktion 75-90 %; 3 = volle Wirkung, Wurmreduktion >90 % | | | | | |

### Herstellungsbeispiele

Beispiele für die Herstellung der cyclischen Depsipeptide mit 24 Ringatomen:
1. Herstellung der Verbindungen der Formel (I).
   Zu einer Lösung der Verbindung der Formel II (0,104 mmol) und Hünig-Base (0,258 mmol) in Dichlormethan (100 ml) wurde bei 0°C BOP-Cl (0,124 mmol) zugegeben und 24 h bei Raumtemperatur nachgerührt. Nach dieser Zeit wurden dieselben Mengen BOP-Cl und Base zugesetzt und weitere 24 h gerührt. Die Lösung wurde zweimal mit ges. Natriumhydrogencarbonatlösung gewaschen, über Natriumsulfat getrocknet und eingeengt. Der Rückstand wurde säulenchromatografisch mit dem Laufmittel Cyclohexan-Ethylacetat 2:1 gereinigt.
   Es wurden Verbindungen der Formel (I) erhalten, in welcher die Substituenten die folgende Bedeutung haben (Tabelle 3):

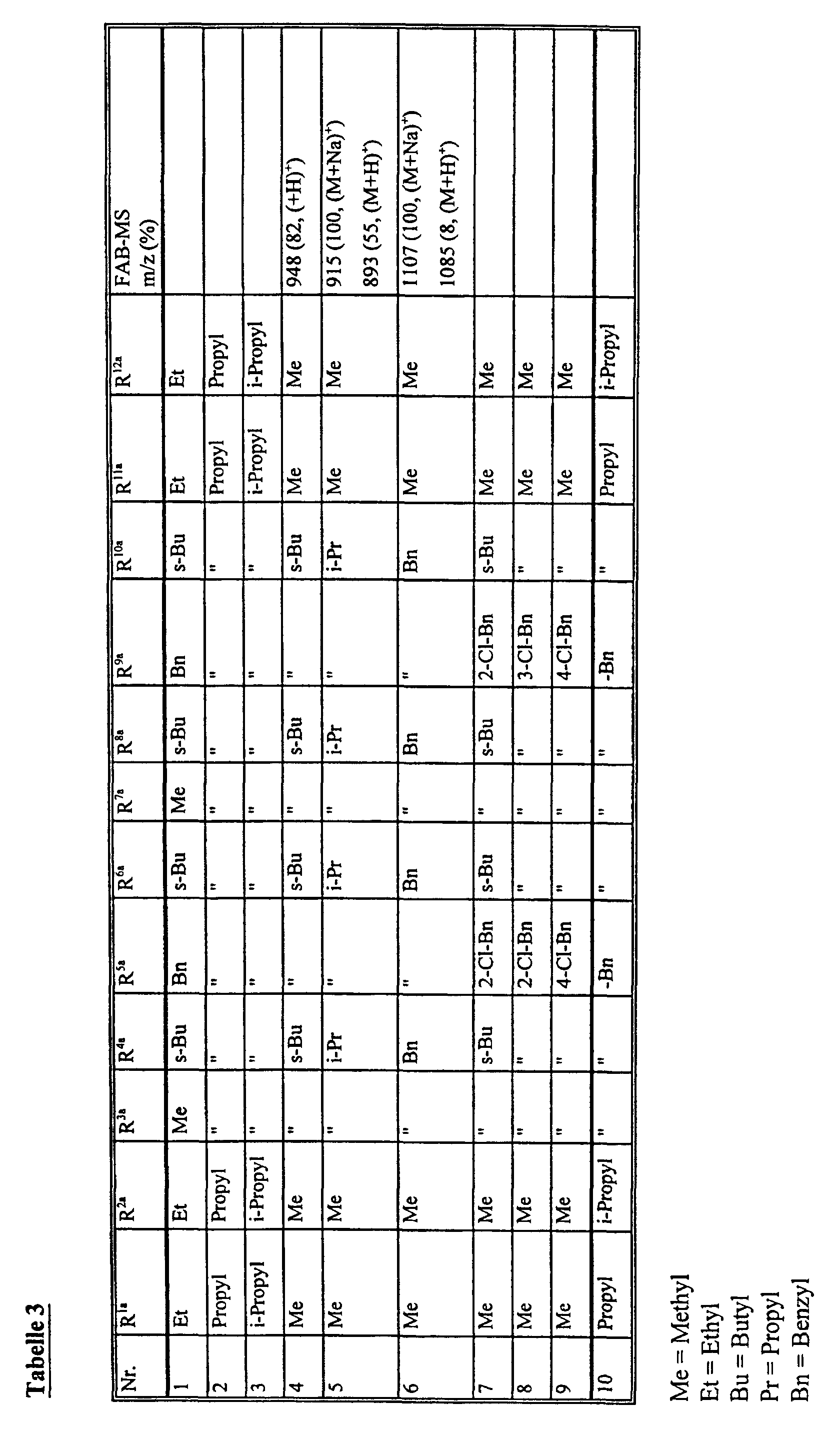

### Beispiele für die Herstellung der Verbindungen der Formel (II)

Eine Lösung eines offentkettigen Octadepsipeptides der Formel (III) (1,222 mmol) in Ethanol (50 ml) wurde in Gegenwart von Pd(OH)₂/C (20 %; 200 mg) bis zur Beendigung der Wasserstoffaufnahme hydriert (ca. 2 h). Nach Abfiltrieren des Katalysators fiel reine Verbindung der Formel II an, die ohne zusätzliche Reinigung weiter umgesetzt wurde.

Gemäß dieser Vorschrift wurden Verbindungen der Formel (II) erhalten, in welcher die Substituenten die Bedeutung gemäß Tabelle 4 haben.

**Tabelle 4**

| Nr. | R^{1a} | R^{2a} | R^{3a} | R^{4a} | R^{5a} | R^{6a} | R^{7a} | R^{8a} | R^{9a} | R^{10a} | R^{11a} | R^{12a} |
|---|---|---|---|---|---|---|---|---|---|---|---|---|
| 11 | Et | Et | Me | s-Bu | Bn | s-Bu | Me | s-Bu | Bn | s-Bu | Et | Et |
| 12 | Propyl | Propyl | " | " | " | " | " | " | " | " | Propyl | Propyl |
| 13 | i-Propyl | i-Propyl | " | " | " | " | " | " | " | " | i-Propyl | i-Propyl |
| 14 | Me | Me | " | " | " | " | " | " | " | " | Me | Me |
| 15 | Me | Me | " | i-Pr | " | i-Pr | " | i-Pr | " | i-Pr | Me | Me |
| 16 | M4 | Me | " | Bn | " | Bn | " | Bn | " | Bu | Me | Me |
| 17 | Me | Me | " | s-Bu | 2-Cl- | s-Bu | " | s-Bu | 2-Cl-Bn | s-Bu | Me | Me |
| | | | | | Bn | | | | | | | |
| 18 | Me | Me | " | " | 3-Cl- | " | " | " | 3-Cl-Bn | " | Me | Me |
| | | | | | Bn | | | | | | | |
| 19 | Me | Me | " | " | 4-Cl- | " | " | " | 4-Cl-Bn | " | Me | Me |
| | | | | | Bn | | | | | | | |
| 20 | Propyl | i-Propyl | " | " | -Bn | " | " | " | -Bn | " | Propyl | i-Propyl |
| Me = Methyl Et = Ethyl s-Bu = s-Butyl Bn = Benzyl | | | | | | | | | | | | |

### Herstellung der Verbindungen der Formel (III)

In eine Lösung des tert.-Butylesters der Formel (IV) (1.609 mmol) in Dichlormethan (40 ml) wurde bei 0°C 1,5 h HCl-Gas eingeleitet. Anschließend wurde auf Raumtemperatur aufgewärmt und 12 h nachgerührt. Die Lösung wurde einrotiert und im Hochvakuum getrocknet. Der Rückstand wurde ohne weitere Reinigung umgesetzt.

Analog wurden Verbindungen der Formel (III) erhalten, in welcher die Substituenten die folgende Bedeutung haben (Tabelle 5):

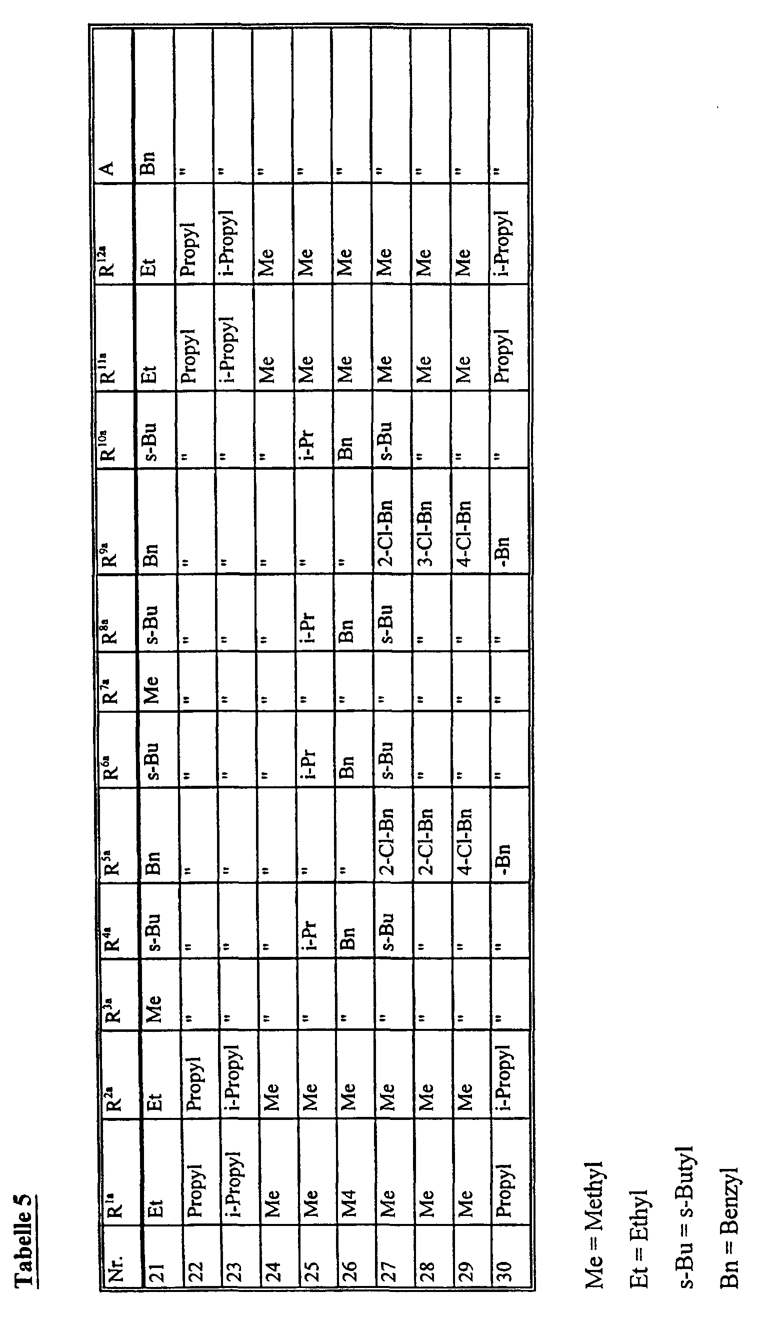

### Herstellung der Verbindungen der Formel (IV)

Zu einer Lösung der Tetradepsipeptide der Formel (VI) und (V) je (2.52 mmol), in Dichlormethan (15 ml) wurde bei 0°C eine Lösung von Ethyldiisopropylamin (0,912 mmol) und BOP-Cl (0,438 mmol) zugegeben. Es wurde 1 h bei 0°C und 1,5 h bei Raumtemperatur nachgerührt, mit 20 ml Dichlormethan verdünnt, zweimal mit wenig Wasser gewaschen, über Na₂SO₄ getrocknet und eingeengt. Der Rückstand wurde an Kieselgel mit dem Laufmittel Cyclohexan-t-BuOMe = 2 : 1 gereinigt.

### Herstellung der Verbindungen der Formel (V)

In eine Lösung des Tetradepsipeptids mit der Formel (VII) (2.848 mmol) in Dichlormethan (50 ml) wurde bei 0°C HCl-Gas 2 h eingeleitet.

Anschließend wurde 8 h bei Raumtemperatur nachgerührt, eingeengt und im Hochvakuum getrocknet. Der Rückstand wurde ohne weitere Reinigung eingesetzt.

### Herstellung der Verbindungen der Formel (VI)

Eine Lösung des Tetradepsipeptids mit der Formel (VII) (9.53 mmol) in Ethanol (37 ml) wurde mit Pd(OH)₂/C (20 %) (0,6 g) versetzt und ca. 3 h bei Raumtemperatur und Normaldruck hydriert. Das Reaktionsgemisch wurde filtriert, eingeengt und der Rückstand an Kieselgel mit dem Laufmittel t-BuOMe-Cyclohexan-Ethanol = 1:1:0,5 getrennt.

### Herstellung der Verbindungen der Formel (VII)

Eine auf 0°C gekühlte Lösung des Didepsipeptids IX (22,9 mmol) und des Didepsipeptids VIIIa (27,5 mmol) in Dichlormethan (80 ml) wurde mit Diisopropylethylamin (57,3 mmol) und BOP-Cl (29,8 mmol) versetzt, 1 h bei 0°C und 1 h bei Raumtemperatur gerührt. Nach Abfiltrieren des Niederschlages wurde die Lösung mit Dichlormethan verdünnt, dreimal mit wenig Wasser gewaschen, über Natriumsulfat getrocknet und eingeengt. Der Rückstand wurde an Kieselgel mit dem Laufmittel Cyclohexan-Ethylacetat = 15:1 getrennt.

## Patentansprüche

1. Endoparasitizide Mittel, die Piperazine zusammen mit cyclischen Depsipeptiden bestehend aus Aminosäuren und Hydroxycarbonsäuren als Ringbausteinen und mit 24 Ringatomen enthalten.

2. Verwendung von Piperazinen zusammen mit cyclischen Depsipeptiden bestehend aus Aminosäuren und Hydroxycarbonsäuren als Ringbausteinen und mit 24 Ringatomen zur Herstellung von endoparasitiziden Mitteln.

3. Verwendung von Piperazinen gemäß Anspruche 2, **dadurch gekennzeichnet, daß** die cyclischen Depsipeptide der Formel (I) 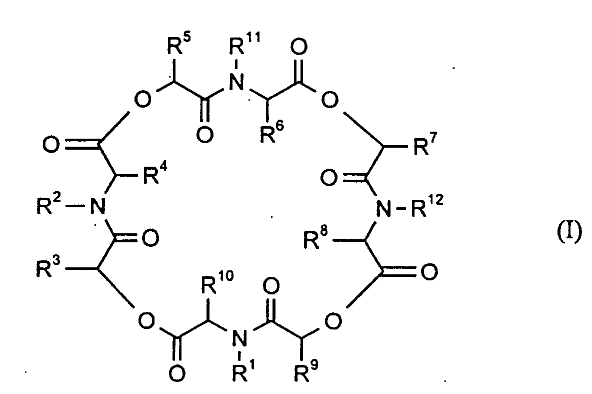 in welcher
R¹, R², R¹¹ und R¹² unabhängig voneinander für C₁₋₈-Alkyl, C₁₋₈-Halogenalkyl, C₃₋₆-Cycloalkyl, Aralkyl, Aryl stehen,
R³, R⁵, R⁷, R⁹ unabhängig voneinander für Wasserstoff oder geradkettiges oder verzweigtes C₁₋₈-Alkyl steht, das gegebenenfalls durch Hydroxy, C₁₋₄-Alkoxy, Carboxy, 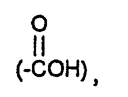 Carboxamid, 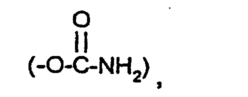 Imidazolyl, Indolyl,
Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
R⁴, R⁶, R⁸, R¹⁰ unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen
sowie deren optische Isomere und Racemate, entsprechen.

4. Verwendung gemäß Anspruch 3, dadurch gekenzeichnet, daß die cyclischen Depsipeptide der Formel (I) entsprechen, in welcher
R¹, R², R¹¹ und R¹² unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können und
R³ bis R¹⁰ die in Anspruch 3 angegebene Bedeutung haben.

5. Verwendung gemäß Anspruch 3, **dadurch gekennzeichnet, daß** die cyclischen Depsipeptide der Formel (I) entsprechen, in welcher
R¹, R², R¹¹ und R¹² unabhängig voneinander für Methyl, Ethyl, Propyl, Isopropyl oder n-, s-, t-Butyl stehen,
R³, R⁵, R⁷, R⁹ für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können und
R⁴, R⁶, R⁸, R¹⁰ unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können sowie für Isopropyl, s-Butyl ferner für gegebenenfalls halogensubstituiertes Phenyl, Benzyl oder Phenylethyl stehen.

6. Verwendung gemäß Ansprüchen 2 bis 5, **dadurch gekennzeichnet, daß** die Piperazine der Formel (X) entsprechen, 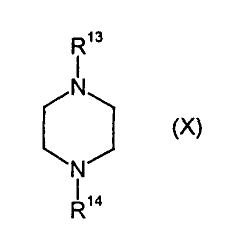 in welcher
R¹³ und R¹⁴ unabhängig voneinander für gleiche oder verschiedene Substituenten aus der Gruppe Wasserstoff, jeweils gegebenenfalls substituiertes Alkyl, Cycloalkyl, Aryl, Heteroaryl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, jeweils gegebenenfalls substituiertes Alkyl oder Cycloalkyl stehen.

7. Verwendung gemäß Ansprüchen 2 bis 6, **dadurch gekennzeichnet, daß** die Piperazine der Formel (X) entsprechen, in welcher
R¹³ und R¹⁴ unabhängig voneinander für gleiche oder verschiedene Substitüenten aus der Gruppe Wasserstoff, jeweils gegebenenfalls substituiertes C₁-C₆-Alkyl, C₃-C₈-Cycloalkyl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, jeweils gegebenenfalls substituiertes C₁-C₆-Alkyl oder C₃-C₈-Cycloalkyl stehen.

8. Verwendung gemäß Ansprüchen 2 bis 7, **dadurch gekennzeichnet, daß** die Piperazine der Formel (X) entsprechen, in welcher
R¹³ und R¹⁴ unabhängig voneinander für gleiche oder verschiedene Substituenten aus der Gruppe Wasserstoff, jeweils gegebenenfalls substituiertes C₁-C₄-Alkyl, C₆-Cycloalkyl sowie -CONR¹⁵R¹⁶ oder -CSNR¹⁵R¹⁶ stehen, in welchen
R¹⁵ und R¹⁶ unabhängig voneinander für gleiche oder verschiedene Substituenten aus der Gruppe Wasserstoff, jeweils gegebenenfalls substituiertes C₁-C₄-Alkyl oder C₆-Cycloalkyl stehen.

9. Mittel gemäß Anspruch 1, **dadurch gekennzeichnet, daß** die cyclischen Depsipeptide einer der in Ansprüchen 4 bis 6 genannten Definitionen entsprechen und/oder die Piperazine einer der in den Ansprüchen 7 bis 9 genannten Definitionen entsprechen.

## Claims

1. Endoparasiticidal compositions which contain piperazines together with cyclic depsipeptides consisting of amino acids and hydroxycarboxylic acids as ring units and having 24 ring atoms.

2. Use of piperazines together with cyclic depsipeptides consisting of amino acids and hydroxycarboxylic acids as ring units and having 24 ring atoms for the production of endoparasiticidal compositions.

3. Use of piperazines according to Claim 2, **characterized in that** the cyclic depsipeptides correspond to the formula (I) 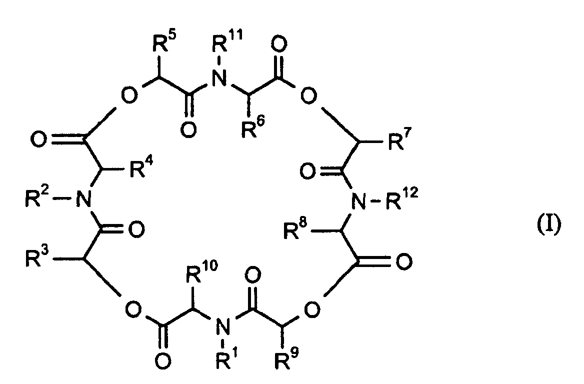 in which
R¹, R², R¹¹ and R¹² independently of one another represent C₁₋₈-alkyl, C₁₋₈-halogenoalkyl, C₃₋₆-cycloalkyl, aralkyl, aryl,
R³, R⁵, R⁷, R⁹ independently of one another represent hydrogen or straight-chain or branched C₁₋₈-alkyl, which can optionally be substituted by hydroxyl, C₁₋₄-alkoxy, carboxyl, 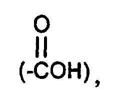 carboxamide, 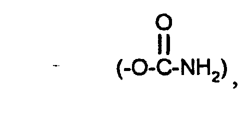 , imidazolyl, indolyl, guanidino, -SH or C₁₋₄-alkylthio and further represent aryl or aralkyl which can be substituted by halogen, hydroxyl, C₁₋₄-alkyl, C₁₋₄-alkoxy,
R⁴, R⁶, R⁸, R¹⁰ independently of one another represent hydrogen, straight-chain C₁₋₅-alkyl, C₂₋₆-alkenyl, C₃₋₇-cycloalkyl, each of which can optionally be substituted by hydroxyl, C₁₋₄-alkoxy, carboxyl, carboxamide, imidazolyl, indolyl, guanidino, SH or C₁₋₄-alkylthio, and represent aryl or aralkyl which can be substituted by halogen, hydroxyl, C₁₋₄-alkyl, C₁₋₄-alkoxy,
and their optical isomers and racemates.

4. Use according to Claim 3, **characterized in that** the cyclic depsipeptides correspond to the formula (I), in which
R¹, R², R¹¹ and R¹² independently of one another represent methyl, ethyl, propyl, isopropyl, n-, s-, t-butyl or phenyl, which is optionally substituted by halogen, C₁₋₄-alkyl, OH, C₁₋₄-alkoxy, and also represent benzyl or phenethyl, each of which can optionally be substituted by the radicals indicated in the case of phenyl, and
R³ to R¹⁰ have the meaning indicated in claim 3.

5. Use according to Claim 3, **characterized in that** the cyclic depsipeptides correspond to the formula (I), in which
R¹, R², R¹¹ and R¹² independently of one another represent methyl, ethyl, propyl, isopropyl or n-, s-, t-butyl,
R³, R⁵, R⁷, R⁹ represent hydrogen, straight-chain or branched C₁₋₈-alkyl, in particular methyl, ethyl, propyl, i-propyl, n-, s-, t-butyl, each of which can optionally be substituted by C₁₋₄-alkoxy, in particular methoxy, ethoxy, imidazolyl, indolyl or C₁₋₄-alkylthio, in particular methylthio, ethylthio, and further respresent phenyl, benzyl or phenethyl, each of which can optionally be substituted by halogen, in particular chlorine, and
R⁴, R⁶, R⁸, R¹⁰ independently of one another represent hydrogen, methyl, ethyl, n-propyl, n-butyl, vinyl, cyclohexyl, each of which can optionally be substituted by methoxy, ethoxy, imidazolyl, indolyl, methylthio, ethylthio, and represent isopropyl, s-butyl and further represent optionally halogen-substituted phenyl, benzyl or phenylethyl.

6. Use according to Claims 2 to 5, **characterized in that** the piperazines correspond to the formula (X), 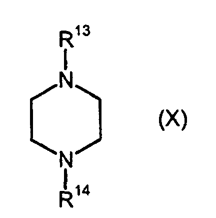 in which
R¹³ and R¹⁴ independently of one another represent identical or different substituents of the group hydrogen, in each case optionally substituted alkyl, cycloalkyl, aryl, heteroaryl, and -CONR¹⁵R¹⁶ or -CSNR¹⁵R¹⁶ , in which
R¹⁵ and R¹⁶ independently of one another represent identical or different substituents of the group hydrogen, in each case optionally substituted alkyl or cycloalkyl.

7. Use according to Claims 2 to 6, **characterized in that** the piperazines correspond to the formula (X), in which
R¹³ and R¹⁴ independently of one another represent identical or different substituents of the group hydrogen, in each case optionally substituted C₁-C₆-alkyl, C₃-C₈-cycloalkyl, and -CONR¹⁵R¹⁶ or - CSNR¹⁵R¹⁶, in which
R¹⁵ and R¹⁶ independently of one another represent identical or different substituents of the group hydrogen, in each case optionally substituted C₁-C₆-alkyl or C₃-C₈-cycloalkyl.

8. Use according to Claims 2 to 7, **characterized in that** the piperazines correspond to the formula (X), in which
R¹³ and R¹⁴ independently of one another represent identical or different substituents of the group hydrogen, in each case optionally substituted C₁-C₄-alkyl, C₆-cycloalkyl, and -CONR¹⁵R¹⁶ or - CSNR¹⁵R¹⁶, in which
R¹⁵ and R¹⁶ independently of one another represent identical or different substituents of the group hydrogen, in each case optionally substituted C₁-C₄-alkyl or C₆-cycloalkyl.

9. Composition according to Claim 1, **characterized in that** the cyclic depsipeptides correspond to one of the definitions mentioned in claims 3 to 5 and/or the piperazines correspond to one of the definitions mentioned in claims 6 to 8.

## Revendications

1. Compositions endoparasiticides, qui contiennent des pipérazines conjointement avec des depsipeptides cycliques constitués d'amino-acides et d'acides hydroxycarboxyliques comme éléments constitutifs du noyau et comportant 24 atomes dans le cycle.

2. Utilisation de pipérazines conjointement avec des depsipeptides cycliques constitués d'amino-acides et d'acides hydroxycarboxyliques comme éléments constitutifs du noyau et comportant 24 atomes dans le cycle pour la préparation de compositions endoparasiticides.

3. Utilisation de pipérazines suivant la revendication 2, **caractérisée en ce que** les depsipeptides cycliques de formule (I) répondent à la formule 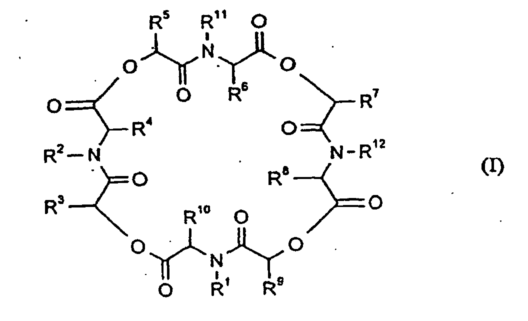 dans laquelle
R¹, R², R¹¹ et R¹² représentent, indépendamment les uns des autres, un reste alkyle en C₁ à C₈, halogénalkyle en C₁ à C₈, cycloalkyle en C₃ à C₆, aralkyle, aryle,
R³, R⁵, R⁷, R⁹ représentent, indépendamment les uns des autres, l'hydrogène ou un reste alkyle en C₁ à C₈ linéaire ou ramifié qui peut être substitué, le cas échéant, par un radical hydroxy, alkoxy en C₁ à C₄, carboxy, 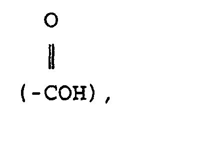 carboxamide, 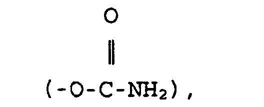 imidazolyle, indolyle, guanidino, -SH ou alkylthio en C₁ à C₄ et représente en outre un reste aryle ou un reste aralkyle qui peuvent être substitués par un radical halogéno, hydroxy, alkyle en C₁ à C₄, alkoxy en C₁ à C₄,
R⁴, R⁶, R⁸, R¹⁰ représentent, indépendamment les uns des autres, un reste alkyle linéaire en C₁ à C₅, un reste alcényle en C₂ à C₆, un reste cycloalkyle en C₃ à C₇, qui peuvent éventuellement être substitués par un radical hydroxy, alkoxy en C₁ à C₄, carboxy, carboxamide, imidazolyle, indolyle, guanidino, SH ou alkylthio en C₁ à C₄, ainsi qu'un reste aryle ou un reste aralkyle qui peuvent être substitués par un radical halogéno, hydroxy, alkyle en C₁ à C₄, alkoxy en C₁ à C₄,
ainsi que leurs isomères optiques et leurs racémates.

4. Utilisation suivant la revendication 3, **caractérisée en ce que** les depsipeptides cycliques répondent à la formule (I) dans laquelle
R¹, R², R¹¹ et R¹² représentent, indépendamment les uns des autres, un reste méthyle, éthyle, propyle, isopropyle, n-butyle, sec-butyle, tertio-butyle ou un reste phényle qui est éventuellement substitué par un radical halogéno, alkyle en C₁ à C₄, OH, alkoxy en C₁ à C₄, ainsi qu'un reste benzyle ou un reste phényléthyle qui peuvent être substitués, le cas échéant, par les radicaux indiqués pour le reste phényle, et
R³ à R¹⁰ ont la définition indiquée dans la revendication 3 .

5. Utilisation suivant la revendication 3, **caractérisée en ce que** les depsipeptides cycliques répondent à la formule (I), dans laquelle
R¹, R², R¹¹ et R¹² représentent, indépendamment les uns des autres, un reste méthyle, éthyle, propyle, isopropyle, n-butyle, sec-butyle ou tertio-butyle,
R³, R⁵, R⁷, R⁹ représentent l'hydrogène, un reste alkyle en C₁ à C₈ linéaire ou ramifié, en particulier les restes méthyle, éthyle, propyle, isopropyle, n-butyle, sec-butyle, tertio-butyle, qui peuvent éventuellement être substitués par un radical alkoxy en C₁ à C₄, notamment méthoxy, éthoxy, imidazolyle, indolyle, ou un radical alkylthio en C₁ à C₄, notamment méthylthio, éthylthio, en outre un reste phényle, un reste benzyle ou un reste phénéthyle, qui peuvent éventuellement être substitués par un halogène, notamment du chlore, et
R⁴, R⁶, R⁸, R¹⁰ représentent, indépendamment les uns des autres, l'hydrogène, les restes méthyle, éthyle, n-propyle, n-butyle, vinyle, cyclohexyle, qui peuvent éventuellement être substitués par un radical méthoxy, éthoxy, imidazolyle, indolyle, méthylthio, éthylthio, ainsi qu'un reste isopropyle, sec-butyle, en outre un reste phényle, benzyle ou phényléthyle qui est éventuellement substitué par un halogène.

6. Utilisation suivant les revendications 2 à 5, **caractérisée en ce que** les pipérazines répondent à la formule (X) 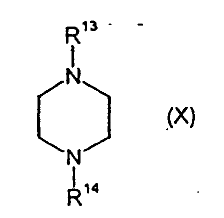 dans laquelle
R¹³ et R¹⁴ représentent, indépendamment les uns des autres, des substituants identiques ou différents du groupe de l'hydrogène, de radicaux alkyle, cycloalkyle, aryle, hétéroaryle, dont chacun est éventuellement substitué, ainsi que de radicaux -CONR¹⁵R¹⁶
ou -CSNR¹⁵R¹⁶, dans lesquels
R¹⁵ et R¹⁶ représentent indépendamment l'un de l'autre des substituants identiques ou différents du groupe de l'hydrogène, de radicaux alkyle ou cycloalkyle dont chacun est éventuellement substitué.

7. Utilisation suivant les revendications 2 à 6, **caractérisée en ce que** les pipérazines répondent à la formule (X) dans laquelle
R¹³ et R¹⁴ représentent, indépendamment les uns des autres, des substituants identiques ou différents du groupe de l'hydrogène, de radicaux alkyle en C₁ à C₆, cycloalkyle en C₃ à C₈ dont chacun est éventuellement substitué, ainsi que des radicaux -CONR¹⁵R¹⁶ ou -CSNR¹⁵R¹⁶, dans lesquels
R¹⁵ et R¹⁶ représentent, indépendamment les uns des autres, des substituants identiques ou différents du groupe de l'hydrogène et de radicaux alkyle en C₁ à C₆ ou cycloalkyle en C₃ à C₈ dont chacun est éventuellement substitué.

8. Utilisation suivant les revendications 2 à 7, **caractérisée en ce que** les pipérazines répondent à la formule (X), dans laquelle
R¹³ et R¹⁴ représentent, indépendamment les uns des autres, des substituants identiques ou différents du groupe de l'hydrogène, de radicaux alkyle en C₁ à C₄, cycloalkyle en C₆, dont chacun est éventuellement substitué, ainsi que de radicaux -CONR¹⁵R¹⁶ ou -CSNR¹⁵R¹⁶, dans lesquels
R¹⁵ et R¹⁶ représentent indépendamment l'un de l'autre des substituants. identiques ou différents du groupe de l'hydrogène et de radicaux alkyle en C₁ à C₄ ou cycloalkyle en C₆ dont chacun est éventuellement substitué.

9. Compositions suivant la revendication 1, **caractérisée en ce que** les depsipeptides cycliques répondent à l'une des définitions mentionnées dans les revendications 4 à 6 et/ou les pipérazines répondent à l'une des définitions indiquées dans les revendications 7 à 9.
